# EUROPEAN PATENT SPECIFICATION

(11) **EP 4 518 799 B1**
(45) Date of publication and mention of the grant of the patent: **04.11.2026**
(21) Application number: 23725784.5
(22) Date of filing: 26.04.2023
(51) Int. Cl.: A61F 2/24

(54) **TRANSCATHETER DELIVERY DEVICE HAVING FLEXIBLE CAPSULE**
TRANSKATHETEREINFÜHRUNGSVORRICHTUNG MIT FLEXIBLER KAPSEL
DISPOSITIF D'ADMINISTRATION PAR TRANSCATHÉTER AYANT UNE CAPSULE FLEXIBLE

(30) Priority: 03.05.2022 US 202263337755 P
(43) Date of publication of application: 12.03.2025
(73) Proprietor: Medtronic, Inc., Minneapolis, MN 55432 (US)
(72) Inventor: NIGADE, Anish S., Minneapolis, Minnesota 55432 (US); PRIMEAUX, Jonathan, Minneapolis, Minnesota 55432 (US)
(74) Representative: Zimmermann & Partner Patentanwälte mbB
(86) International application number: PCT/IB2023/054305
(87) International publication number: WO 2023/214253

(56) References cited:
- US-A1- 2017 128 205
- US-A1- 2018 098 848

## Description

### FIELD

The present technology is generally related to transcatheter delivery devices and methods for delivering a cardiac prosthesis. More particularly, the present technology is related to transcatheter delivery devices having a flexible capsule for at least partially sheathing the cardiac prosthesis.

### BACKGROUND

A human heart includes four heart valves that determine the pathway of blood flow through the heart: the mitral valve, the tricuspid valve, the aortic valve, and the pulmonary valve. The mitral and tricuspid valves are atrio-ventricular valves, which are between the atria and the ventricles, while the aortic and pulmonary valves are semilunar valves, which are in the arteries leaving the heart. Ideally, native leaflets of a heart valve move apart from each other when the valve is in an open position, and meet or "coapt" when the valve is in a closed position. Problems that may develop with valves include stenosis in which a valve does not open properly, and/or insufficiency or regurgitation in which a valve does not close properly. Stenosis and insufficiency may occur concomitantly in the same valve. The effects of valvular dysfunction vary, with regurgitation or backflow typically having relatively severe physiological consequences to the patient.

Diseased or otherwise deficient heart valves can be repaired or replaced using a variety of different types of heart valve surgeries. One conventional technique involves an open-heart surgical approach that is conducted under general anesthesia, during which the heart is stopped and blood flow is controlled by a heart-lung bypass machine.

More recently, minimally invasive approaches have been developed to facilitate catheter-based implantation of a prosthetic heart valve or prosthesis on the beating heart, intending to obviate the need for the use of classical sternotomy and cardiopulmonary bypass. In general terms, an expandable prosthetic valve is compressed about or within a catheter, inserted inside a body lumen of the patient, such as the femoral artery, and delivered to a desired location in the heart.
US 2017/128205 A1 describes an implant delivery capsule.

The heart valve prosthesis employed with catheter-based, or transcatheter, procedures generally includes an expandable multi-level frame or stent that supports a valve structure having a plurality of leaflets. The frame can be contracted during percutaneous transluminal delivery, and expanded upon deployment at or within the native valve. One type of valve stent can be initially provided in an expanded or uncrimped condition, then crimped or compressed about a balloon portion of a catheter. The balloon is subsequently inflated to expand and deploy the prosthetic heart valve. With other stented prosthetic heart valve designs, the stent frame is formed to be self-expanding. With these systems, the valved stent is crimped down to a desired size and held in that compressed state within a sheath for transluminal delivery. Retracting the sheath from this valved stent allows the stent to self-expand to a larger diameter, fixating at the native valve site. In more general terms, then, once the prosthetic valve is positioned at the treatment site, for instance within an incompetent native valve, the stent frame structure may be expanded to hold the prosthetic valve firmly in place.

The present disclosure addresses problems and limitations associated with the related art.

### SUMMARY

The present invention is defined in appended claim 1. Preferred embodiments of the invention are defined in the dependent claims.

The techniques of this disclosure generally relate to transcatheter delivery devices and methods for delivery and deployment of a cardiac prosthesis, such as a prosthetic heart valve. Various embodiments include a flexible capsule for at least partially sheathing the prosthesis during delivery. In various examples, a flexible, distal capsule is used in conjunction with a second, proximal capsule to sheathe the prosthesis during delivery. In various examples, the distal capsule can be forced into a generally straightened state and transition to a generally curved or first state to provide clearance during delivery and deployment of the prosthesis. Alternatively, the distal capsule can be configured to otherwise deflect upon contact with the anatomy. Embodiments of the disclosure can reduce the ventricle depth of the delivery device during deployment of the prosthesis, which increases the patient population and valve locations suitable for prosthesis delivery with the delivery device.

According to claim 1, the present invention provides a system including a delivery device having an outer sheath having a rigid distal portion and an inner sheath positioned at least partially within the outer sheath. The inner sheath has a distal end. The delivery device also includes a shaft coaxially positioned at least partially within the outer sheath and the inner sheath. The delivery device includes a flexible capsule connected to a distal end of the shaft. The flexible capsule at least partially defines a prosthesis compartment and the flexible capsule has a first state in which a longitudinal axis of the flexible capsule is curved. The delivery device has a second state in which the flexible capsule is positioned within the distal portion of the outer sheath such that the rigidity of the distal portion of the outer sheath reduces the curvature of the flexible capsule. The delivery device further has a state in which the flexbile capsule is positioned outside of the outer sheath in the first state.

In one aspect, the present disclosure provides a system including a delivery device having an outer sheath having a rigid distal portion. The delivery device also includes an inner sheath positioned at least partially within the outer sheath. The inner sheath has a distal end. The delivery device additionally includes a proximal capsule connected the distal end of the inner sheath. A shaft is coaxially positioned at least partially within the outer sheath and the inner sheath. A distal capsule is connected to a distal end of the shaft. The proximal capsule and the distal capsule collectively define a prosthesis compartment. The distal capsule has a generally curved state in which a longitudinal axis of the distal capsule is curved, defining an angle, and the delivery device has a generally straightened, second state in which the distal capsule is positioned within the distal portion of the outer sheath such that the rigidity of the distal portion straightens in the distal capsule from its generally curved state. The delivery device further having a state in which the distal capsule is positioned outside of the outer sheath in the generally curved state.

In yet another aspect, the disclosure provides a method of delivering a prosthesis to a target site within a heart. The method includes providing a delivery device having an outer sheath having a rigid distal portion. The delivery device further including an inner sheath positioned at least partially within the outer sheath. The inner sheath having a distal end. The delivery device has a shaft coaxially positioned at least partially within the outer sheath and the inner sheath. Additionally, the delivery device includes a flexible capsule connected to a distal end of the shaft. The flexible capsule at least partially defines a prosthesis compartment. A prosthetic heart valve is positioned on the shaft and within the prosthesis compartment. The method includes delivering the prosthesis to a heart valve and positioning the flexible capsule out of the outer sheath and into a ventricle of the heart such that the flexible capsule transitions to a first state having a curved longitudinal axis once advanced out of the outer sheath.

In another aspect, the disclosure provides methods of delivering a prosthesis to a target site within a heart. Such methods include providing a delivery device. The delivery device has an outer sheath having a rigid distal portion and the delivery device also has an inner sheath positioned at least partially within the outer sheath. The inner sheath has a distal end. The delivery device additionally includes a proximal capsule connected the distal end of the inner sheath. A shaft is coaxially positioned at least partially within the outer sheath and the inner sheath. The delivery device further includes a distal capsule connected to a distal end of the shaft. The proximal capsule and the distal capsule collectively define a prosthesis compartment. A prosthetic heart valve is positioned on the shaft and within the prosthesis compartment. The method further includes delivering the prosthesis to a heart valve and then positioning the distal capsule out of the outer sheath and into a ventricle of the heart to separate the distal capsule from the proximal capsule. This action causes the distal capsule to transition to a generally curved state having a generally curved longitudinal axis once advanced out of the outer sheath.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

FIG. 1A is a partial, top view of a delivery device for transcatheter delivery of a prosthesis, the delivery device having a proximal capsule and a distal capsule cooperatively defining a prosthesis compartment.
FIG. 1B is a cross-sectional view of an alternate distal capsule configuration.
FIG. 1C is a cross-sectional view of the distal capsule of FIG. 1A additionally having an inner liner and an outer jacket.
FIG. 2A is a partial, cross-sectional view of the delivery device of FIG. 1 in a closed-capsule configuration or second state.
FIGS. 2B-2C are a partial, cross-sectional views of the delivery device of FIG. 2A with the implant partially deployed.
FIG. 2D is a partial, cross-sectional view of the delivery device of FIGS. 1-2C with the implant deployed.
FIG. 3 is a partial, perspective view of a brim assembly of the delivery device as also shown in FIGS. 2A-2D.
FIG. 4 is a schematic illustration of the delivery device of FIGS. 1A and 2A-2D delivering a prosthesis to a tricuspid valve.
FIG. 5A is a partial, cross-sectional view of an alternate delivery device in a configuration in which the implant is encapsulated for delivery.
FIGS. 5B-5C are partial, cross-sectional view of the delivery device of FIG. 5A with the implant partially deployed.
FIG. 5D is a partial, cross-sectional view of the delivery device of FIGS. 5A-5C with the implant deployed.
FIG. 5E is a partial, cross-sectional view of the delivery device of FIGS. 5A-5D configured for removal from the patient after deployment of the implant.

### DETAILED DESCRIPTION

Specific embodiments of the present disclosure are now described with reference to the figures, wherein like reference numbers indicate identical or functionally similar elements. The terms "distal" and "proximal" are used in the following description with respect to a position or direction relative to the treating clinician. "Distal" or "distally" are a position distant from or in a direction away from the clinician. "Proximal" and "proximally" are a position near or in a direction toward the clinician.

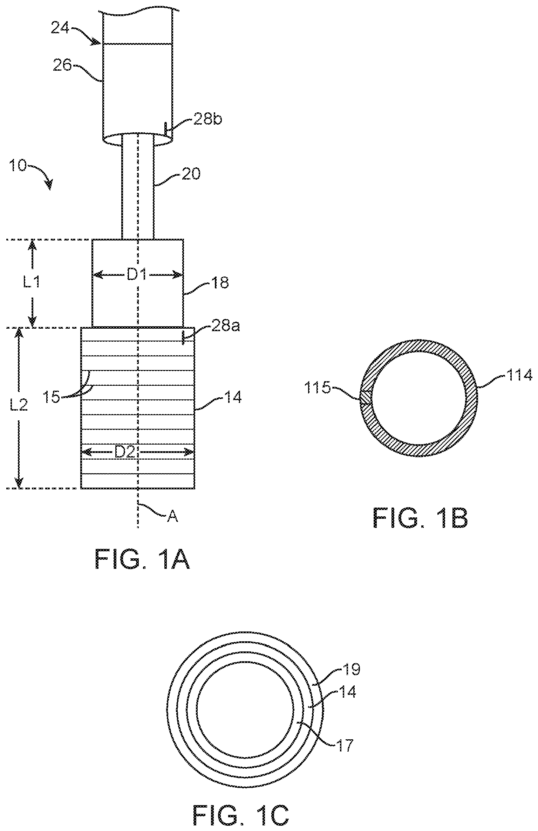

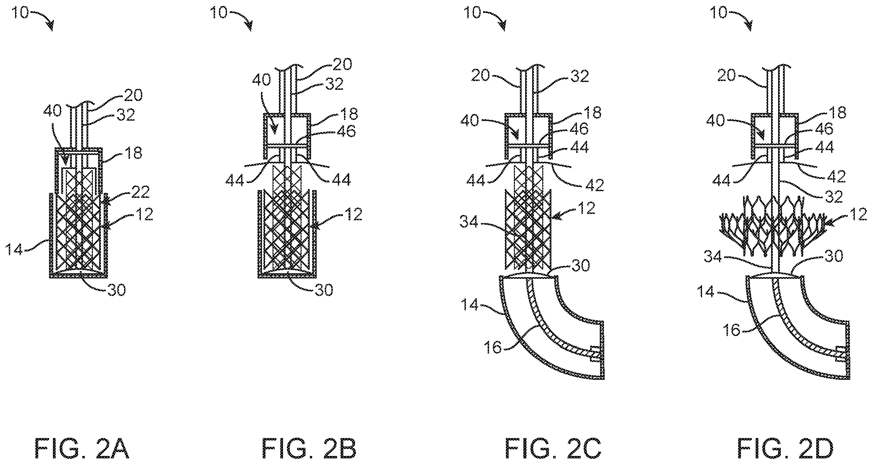

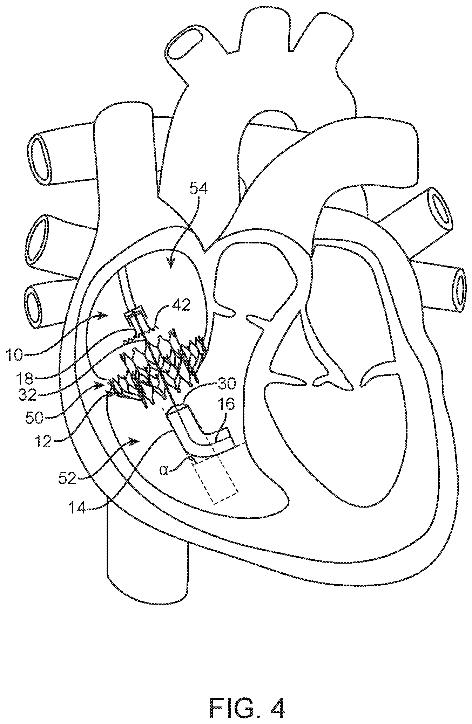

Figures 1A and 2A-2D illustrate a distal end of a delivery device 10 for transcatheter delivery of a cardiac prosthesis 12, such as a prosthetic heart valve. The delivery device 10 includes a distal capsule 14 secured to a shaft 16 and an optional proximal capsule 18 secured to an inner sheath 20 positioned over the shaft 16. Collectively, the proximal capsule 18 and the distal capsule 14 form a prosthesis compartment 22. In some examples, the proximal capsule 18 is omitted and the distal capsule 14 is sized to sheathe a full length of the prosthesis 12. In the illustrated example, the proximal capsule 18 is made of stainless steel or any other biocompatible material and has a smaller diameter D1 than a diameter D2 of the distal capsule 14. In addition, the proximal capsule 18 has a length L1 that is shorter than a length L2 of the distal capsule 14. In one example, the distal and proximal capsules 14, 18 are in a state in which they do not overlap. This state reduces the delivery profile of the loaded capsules 14, 18 compared to configurations in which such capsules do overlap. The prosthesis 12 can be positioned over the shaft 16, compressed and housed within the prosthesis compartment 22 for delivery to a treatment site, such as a heart valve. In various embodiments, the delivery device 10 further includes an outer sheath 24 that can be positioned over the inner shaft 16, the proximal capsule 18 and the distal capsule 14. The outer sheath 24 in some embodiments can include a rigid distal portion 26 that can be positioned to cover the distal capsule 14 and sized so that a length of the rigid distal portion 26 is at least as long as the length L2 of the distal capsule 14. In some examples, the distal capsule 14 is flexible and biased to a curved arrangement when free of outside forces. In some embodiments, the distal capsule can form a curve taking a multitude of angles depending on the degree to which the distal capsule is flexed about its longitudinal axis A. In some embodiments, the longitudinal axis A of the distal capsule 14 defines an angle α between 90-170 degrees (see also, FIG. 4) when not subjected to external forces including the prosthesis 12 or sheath 32 or outer sheath 24. This can optionally be accomplished by forming the distal capsule out of a shape memory material. As represented in the example of FIG. 1A, the distal capsule 14 can be a laser cut, metal hypotube or the like including plurality of slits 15 configured to give the distal capsule 14 flexibility to bend along its longitudinal axis A. In addition, if the distal capsule 14 is made of nitinol or another radiopaque material, the distal capsule 14 can additionally act as an imaging landmark to improve spatial awareness.

In the example of FIG. 1B, a distal capsule 114 may be formed of a flexible material, such as a polymer, having a shape memory rib 115 extending along a length of the distal capsule 114. In this example, the shape memory rib 115 (and thus the distal capsule) can be forced into a straightened, linear arrangement for delivery and will spring to its generally curved state when freed from external forces, thus, also transitioning the distal capsule 114 as a whole to an arrangement similar to what is shown in FIG. 2D. It will be understood that, except as explicitly stated, the distal capsule 114 is otherwise equivalent in use and configuration as compared to the distal capsule 14 of FIG. 1A and FIGS. 2A-2D.

As schematically shown in FIG. 1C, one or more of the capsules of the disclosure (e.g., distal capsule 14) can optionally include an inner liner 17 and an outer jacket 19, each of which can be made of a medium durometer material such as polyether block amide. The inner liner 17 aids in deployment of the prosthesis 12 and can help prevent the prosthesis 12 from snagging on the slits 15, when present. Similarly, the outer jacket 19 can cover and slits 15 and provide a smoother outer surface for delivery.

In some embodiments, a flexible, biased distal capsule is advantageous in that the bend formed in the generally curved state can reduce ventricle depth in which the distal capsule advances within a ventricle while maintaining a length of the prosthesis to be covered. A reduction in device ventricle depth during delivery increases the potential patient population and native valves that can be treated with such delivery devices. This can be additionally advantageous, for example, when treating a tricuspid valve, which typically has a relatively shorter ventricle depth (i.e. space within the anatomy for delivery and deployment).

For transcatheter delivery to the target site, it can be advantageous for the distal capsule 14 to be delivered in a straightened (i.e., having a longitudinal axis that is generally linear or generally not curved) arrangement. To straighten the distal capsule 14, in some embodiments, the outer sheath 24 can be advanced so that the rigid distal portion 26 is positioned over the distal capsule 14, thereby forcing the distal capsule into a corresponding, straightened arrangement similar to what is shown in FIG. 2A.

Various examples can also include a piston 30 secured to a distal terminal end of a sheath 32 positioned over shaft 16. In some of these embodiments, the sheath 32 can have a rigid portion 34 along at least part of its length so that, when positioned within the distal capsule 14, the sheath 32 maintains the distal capsule 14 in the straightened, second state as shown in FIGS. 2A-2B. In the second state of FIG. 2A, the piston 30 is within the prosthesis compartment 22 at the distal end of the distal capsule 14. In this arrangement, the rigidity of the rigid portion 34 forces the distal capsule 14 into the straightened, second state of FIG. 2A. When desired, to allow the distal capsule 14 to flex and reduce a ventricle depth of the delivery device 10, the piston 30 can be proximally withdrawn as shown in FIG. 2D, allowing the distal capsule 14 to flex into its generally curved state.

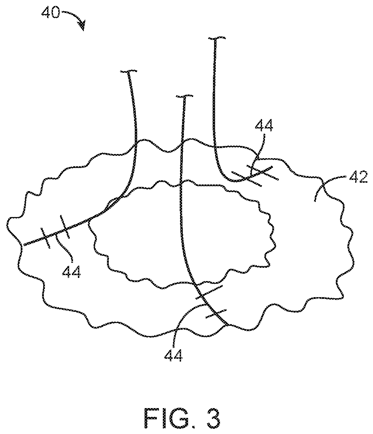

Embodiments of the disclosure can also include an optional brim assembly 40 connected to an interior of the proximal capsule 18 to aid in visualization during a prosthesis deployment procedure from the prosthesis compartment 22. In some such embodiments, the brim assembly 40 is not connected to the prosthesis 12. The brim assembly 40 can include a brim 42 and can optionally include a flexible, fabric material, such as nylon, that circumscribes a circumference of an opening of the proximal capsule 18. The brim 42 can include one or more radiopaque elements 44 that can be viewed under fluoroscopy. In the example of FIG. 3, the brim assembly 40 includes a plurality of radiopaque arms 44 secured to the brim 42 made of a shape memory material, for example. Each arm 44 biases the brim 42 to the flared position of FIG. 3 and is connected to a base 46 that is slidably positioned within the prosthesis compartment 22 within the proximal capsule 18. By incorporating the brim 42 into the capsule 18, as opposed to being directly connected to or incorporated into the prosthesis 12, friction between the crimped prosthesis 12 and the proximal capsule 18 from the prosthesis's radial forces can be reduced in some embodiments, resulting in easier deployment and potential partial or full recapture of the prosthesis 12 within the prosthesis compartment 22 after partial-deployment of the prosthesis.

In yet another example, a distal capsule (e.g., distal capsule 14 of FIG. 1A) is not configured to be biased to a curved arrangement but is flexible so that should the distal capsule interact with patient anatomy during deployment of the prosthesis 12 (such as when the prosthesis is at least partially freed form the distal capsule), the distal capsule deflects upon contact with the anatomy.

A method of deployment of the prosthesis 12 is depicted in FIGS. 2A-2D and FIG. 4. In one method, the delivery device 10 is provided in a delivery arrangement (FIG. 2A) in which the prosthesis 12 is a prosthetic heart valve that is compressed and positioned over and on the sheath 32 (which is over shaft 16) and within the prosthesis compartment 22 with the capsules 14, 18 in the straightened state. In the delivery arrangement, the delivery device 10 delivers the prosthesis 12 to a target site (see also, FIG. 4), which can be a native valve 50. In one example, the delivery device 10 is pushed through a femoral vein (or artery, for example) to reach the inferior vena cava and to a tricuspid valve. In some embodiments including an outer sheath, once the prosthesis 12 is generally in position at the target site, the distal capsule 14 can be advanced out of the outer sheath 24. Alternatively, the outer sheath 24 can be proximally withdrawn to free both the distal and proximal capsules 14, 18.

As shown in FIG. 2B, when ready to start deploying the prosthesis 12, the proximal capsule 18 can be moved proximally to release a proximal end of the prosthesis 12 and the brim 42 (whether the brim is connected to the prosthesis 12 or not) prior to movement of the distal capsule 14. In some examples, the distal capsule 14 can be subsequently moved into the adjacent right or left ventricle 52, or advanced farther into the adjacent right or left ventricle 52.

At the stage of FIG. 2C, the prosthesis 12 can be repositioned or fully or partially recaptured into one or both of the proximal 18 or distal 14 capsules. Once external forces upon it are reduced or removed, the distal capsule 14 can transition to its generally curved state having the curved longitudinal axis A. This may include proximal withdrawal of the piston 30 and its sheath 32. In one example, the distal capsule 14 is delivered so that it will deflect towards the ventricular septum in the generally curved state. To help ensure alignment, corresponding markers or other indicators 28a, 28b (see also FIG. 1A) on one or more of capsules 14, 18 and on the outer sheath 24 will be aligned. This can help ensure that when the outer sheath 24 is articulated, the distal capsule 14 will deflect in the opposite direction. By torquing the shaft 16 or sheath 32, the respective capsules 14, 18 can be rotated with respect the outer sheath 24. By aligning the indicators 28a, 28b on one or more of the capsules 14, 18 and the outer sheath 24 you can ensure that the respective capsule deflects/articulates in the direction opposite (or any other desired direction) of the outer sheath 24 articulation.

As the capsules 14, 18 are separated via movement of respective sheaths/shafts 32, 16, the prosthesis 12 is allowed to expand outside the capsules 14, 18 and at the stage of FIG. 2D, will be ready for deployment from the sheath 32 either via natural expansion or mechanical expansion via a balloon or the like. Generally, the prosthesis 12 is deployed, at least in part, by separating the distal and proximal capsules 14, 18. It will be understood that in embodiments where a proximal capsule is not present, the capsule 18 can simply be distally advanced until the prosthesis 12 is fully unsheathed. The distal capsule 14 can be distally advanced, the proximal capsule 18 can be proximally withdrawn or both the distal and proximal capsules 14, 18 can be separated from each other to free the prosthesis 12 from the confines of the proximal and distal capsules. In some examples, the proximal capsule 18 remains within an atrium 54 adjacent the native valve 50 as the prosthesis 12 is deployed. In some examples, the brim 42 deploys from a position within the proximal capsule 18 to a position at least partially outside of the proximal capsule 18, while being attached to the proximal capsule, as the prosthesis is deployed. Once the prosthesis 12 is deployed from the delivery device 10, the distal capsule 14 can be proximally withdrawn through a center of the prosthesis 12 and removed from the patient in the same manner the delivery device was delivered to the target site.

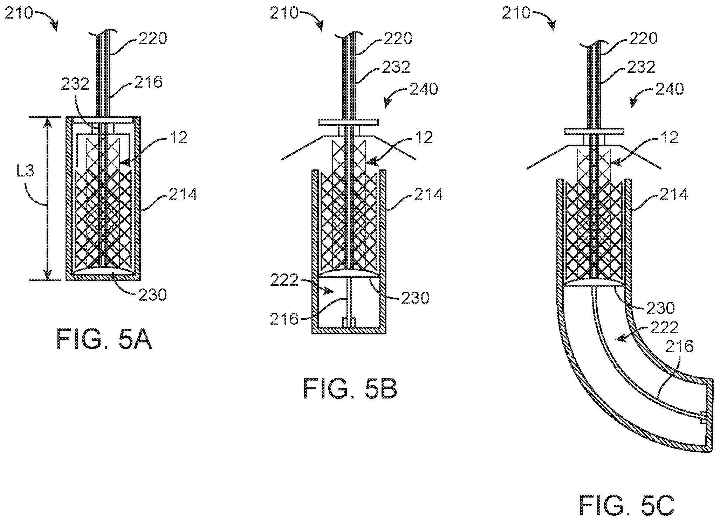

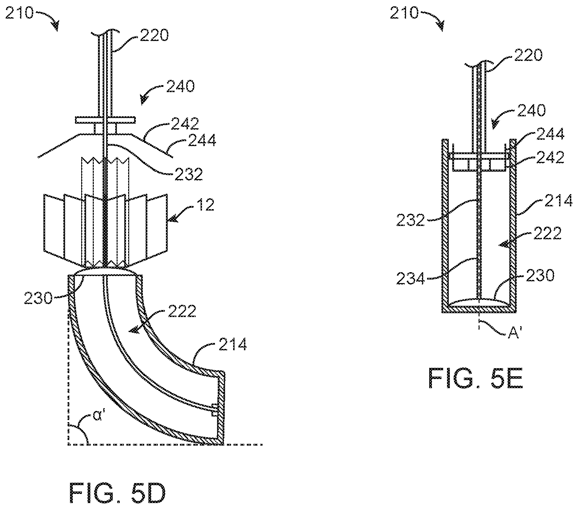

Referring now in addition to FIGS. 5A-5E, which illustrate a distal end of an alternate delivery device 210 for transcatheter delivery of the cardiac prosthesis 12. The delivery device 210 includes a single capsule 214 secured to a shaft 216. Collectively, the capsule 114 forms a prosthesis compartment 222 in which the implant 12 is housed for delivery. The prosthesis 12 can be positioned over the shaft 216, compressed and housed within the prosthesis compartment 222 for delivery to a treatment site, such as a heart valve. In various embodiments, the delivery device 210 further includes an outer sheath 24 (see FIG. 1A) that can be positioned over the inner shaft 216 and the capsule 214. The rigid distal portion 26 of the outer sheath 24 can be positioned to cover the capsule 214 and sized so that a length of the rigid distal portion 26 is at least as long as a length L3 of the capsule 214 similar to that disclosed above with respect to the embodiment of FIG. 1A. In some examples, the capsule 214 is flexible and biased to a curved arrangement when free of outside forces. In some embodiments, the capsule 214 can form a curve taking a multitude of angles depending on the degree to which the capsule is flexed about its longitudinal axis A'. In some embodiments, the longitudinal axis A' of the capsule 214 defines an angle α' between 90-170 degrees when not subjected to external forces including the prosthesis 12 or shaft 216 or outer sheath 24. This can optionally be accomplished by forming the capsule out of a shape memory material. As with prior embodiments, the capsule 214 can be a laser cut, metal hypotube or the like including plurality of slits (see also, FIG. 1A) configured to give the capsule 214 flexibility to bend along its longitudinal axis A' (FIG. 5E). In addition, if the capsule 214 is made of nitinol or another radiopaque material, the distal capsule 214 can additionally act as an imaging landmark to improve spatial awareness. Similar to the example of FIG. 1B, the capsule 214 can alternatively be formed of a flexible material, such as a polymer, having a shape memory rib extending along a length of the distal capsule 214. In this example, the shape memory rib (and thus the capsule) can be forced into a straightened, linear arrangement for delivery and will spring or otherwise naturally transition to its generally curved state when freed from external forces (FIG. 5D). Additionally, the capsule 214 can optionally include an inner liner or outer jacket as discussed with respect to FIG. 1C.

The delivery device 210 can optionally include a brim assembly 240 having a brim 242 and plurality of arms 244, which can be identical to brim assembly 40 except in that it can be secured to inner sheath 220 positioned over the shaft 216 and within the outer sheath 24 (not shown). In this example, the brim assembly 240 is positioned within the capsule 214 until the implant 12 is at least partially deployed (FIGS. 5B-5D). Upon full deployment of the implant 12 (FIG. 5D), the capsule 214 can be proximally withdrawn, to recapture the brim assembly 240. In one example, the arms 244 and brim 242 are inverted during recapture as is shown in FIG. 5E.

Various embodiments of the delivery device 210 can also include a piston 230 secured to a distal terminal end of a sheath 232 positioned over shaft 216. In some of these embodiments, the sheath 232 can have a rigid portion 234 (FIG. 5E) along at least part of its length so that, when positioned within the capsule 214, the sheath 232 at least partially maintains the capsule 214 in the straightened, second state as shown in FIG. 5A. In the state of FIG. 5A, the piston 230 is within the prosthesis compartment 222 at the distal end of the capsule 214. In this arrangement, the rigidity of the rigid portion 234 forces the capsule 214 into the straightened, second state of FIG. 5A. When desired, to allow the capsule 214 to flex and reduce a ventricle depth of the delivery device 210, the piston 230 can be proximally withdrawn as shown in FIG. 5C, allowing the capsule 214 to flex into its generally curved state.

One example of a method of use of the delivery device 210 is generally shown in FIGS. 5A-5E. In FIG. 5A, the crimped implant 12 is positioned within the prosthesis compartment 222 and pushed through a femoral vein to a target site, such as a heart valve. In one example, the capsule 214 is loaded within the outer sheath 24 so that the rigid portion 26 maintains the capsule 214 in the straightened state of FIG. 5A (see also, FIG. 1A). In one example, the distal end of the delivery device 210 is pushed to reach the inferior vena cava and then is directed to a tricuspid valve to position the implant 12 at the tricuspid valve or other target site. Then, the capsule 214 descends into the ventricle, allowing the brim assembly 240, if present, to deploy, which can aid in visualization and positioning of the implant 12 (FIG. 5B). Once desired positioning is achieved, the capsule 214 descends further into the ventricle (FIG. 5C). As the piston 230, sheath 232 and outer sheath 24 are proximal to the capsule 214 at this point, the capsule 214 can either automatically bend and flex to its natural, unbiased arrangement (i.e. predetermined curve) or can flex in response to contact with the anatomy. At the stage of FIG. 5D, the capsule 214 is fully advanced within the ventricle so that the implant 12 is fully outside of the prosthesis compartment 222 and can expand either naturally or mechanically. At this stage, the shaft 216 can be proximally withdrawn to, correspondingly draw the capsule 214 through the implant 12 and recapture the brim assembly 240 within the prosthesis compartment 222 for removal from the patient in the condition of FIG. 5E.

The systems, devices and methods of the disclosure can be used with a variety of cardiac prosthesis types and the present disclosure is not intended to be limited to the prosthesis illustrated in the drawings. Such a prosthesis (e.g., prosthesis 12) can include a bioprosthetic heart valve (not visible for ease of illustration) having tissue leaflets or a synthetic heart valve having polymeric, metallic or tissue-engineered leaflets, and can be specifically configured for replacing valves of the human heart. The stented prosthetic heart valves and other stented prostheses of the present disclosure may be self-expandable, balloon expandable and/or mechanically expandable or combinations thereof. In general terms, the stented prostheses of the present disclosure include a stent or stent frame having an internal lumen maintaining a valve structure (tissue or synthetic), with the stent frame having a normal, expanded condition or arrangement and collapsible to a compressed condition or arrangement for loading within the prosthesis compartment 22 of the delivery device 10. For example, the stents or stent frames are support structures that comprise a number of struts or wire segments arranged relative to each other to provide a desired compressibility and strength to the stented prosthesis. The struts or wire segments are arranged such that they are capable of self-transitioning from, or being forced from, a compressed or collapsed arrangement to a normal, radially expanded arrangement. The struts or wire segments can be formed from a shape memory material, such as a nickel titanium alloy (e.g., nitinol). The stent frame can be laser-cut from a single piece of material, or can be assembled from a number of discrete components.

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

## Claims

1. A system comprising:
a delivery device (10) including:
an outer sheath (24) having a rigid distal portion (26);
an inner sheath (20) positioned at least partially within the outer sheath; the inner sheath having a distal end;
a shaft (16) coaxially positioned at least partially within the outer sheath and the inner sheath;
a flexible capsule (14) connected to a distal end of the shaft; wherein the flexible capsule at least partially defines a prosthesis compartment (22); wherein the flexible capsule has a first state in which a longitudinal axis (A) of the flexible capsule is curved; and
wherein the delivery device has a second state in which the flexible capsule is positioned within the distal portion of the outer sheath such that the rigidity of the distal portion of the outer sheath reduces the curvature of the flexible capsule, the delivery device further having a state in which the flexible capsule is positioned outside of the outer sheath in the first state.

2. The system of claim 1, wherein the flexible capsule is made of a shape memory material biased to the second state.

3. The system of claim 2, wherein the flexible capsule includes a plurality of slits (15).

4. The system of claim 1, wherein the flexible capsule includes an inner liner (17).

5. The system of claim 1, wherein the flexible capsule includes an outer jacket (19).

6. The system of claim 1, further comprising a prosthetic heart valve (12) supported within the flexible capsule.

7. The system of claim 1, further comprising a proximal capsule (18) connected to the distal end of the inner sheath, wherein the flexible capsule and the proximal capsule collectively define the prosthesis compartment.

8. The system of claim 7, wherein the proximal capsule includes a flexible brim (40) extending from a circumference of a distal end of the proximal capsule; wherein the brim includes at least one radiopaque element (44).

9. The system of claim 1, further comprising a piston (30) moveable within the flexible capsule.

10. The system of claim 6, wherein the prosthetic heart valve is a tricuspid valve.

11. The system of claim 8, wherein the brim includes a plurality of arms (44) positioned within the proximal capsule.

12. The system of any preceding claim, wherein the flexible capsule defines an angle of between 90-170 degrees in the first state.

## Patentansprüche

1. System, umfassend:
eine Abgabevorrichtung (10), einschließlich:
einer äußeren Hülle (24), die einen steifen distalen Abschnitt (26) aufweist;
einer inneren Hülle (20), die mindestens teilweise innerhalb der äußeren Hülle positioniert ist; wobei die innere Hülle ein distales Ende aufweist;
eines Schafts (16), der koaxial mindestens teilweise innerhalb der äußeren Hülle und der inneren Hülle positioniert ist;
einer flexiblen Kapsel (14), die mit einem distalen Ende des Schafts verbunden ist; wobei die flexible Kapsel mindestens teilweise eine Prothesenkammer (22) definiert; wobei die flexible Kapsel einen ersten Zustand aufweist, in dem eine Längsachse (A) der flexiblen Kapsel gekrümmt ist; und
wobei die Abgabevorrichtung einen zweiten Zustand aufweist, in dem die flexible Kapsel innerhalb des distalen Abschnitts der äußeren Hülle positioniert ist, sodass die Steifigkeit des distalen Abschnitts der äußeren Hülle die Krümmung der flexiblen Kapsel reduziert, wobei die Abgabevorrichtung ferner einen Zustand aufweist, in dem die flexible Kapsel in dem ersten Zustand außerhalb der äußeren Hülle positioniert ist.

2. System nach Anspruch 1, wobei die flexible Kapsel aus einem Formgedächtnismaterial hergestellt ist, das in den zweiten Zustand vorgespannt ist.

3. System nach Anspruch 2, wobei die flexible Kapsel eine Vielzahl von Schlitzen (15) einschließt.

4. System nach Anspruch 1, wobei die flexible Kapsel eine innere Auskleidung (17) einschließt.

5. System nach Anspruch 1, wobei die flexible Kapsel eine äußere Ummantelung (19) einschließt.

6. System nach Anspruch 1, ferner umfassend eine Herzklappenprothese (12), die innerhalb der flexiblen Kapsel getragen ist.

7. System nach Anspruch 1, ferner umfassend eine proximale Kapsel (18), die mit dem distalen Ende der inneren Hülle verbunden ist, wobei die flexible Kapsel und die proximale Kapsel gemeinsam die Prothesenkammer definieren.

8. System nach Anspruch 7, wobei die proximale Kapsel einen flexiblen Rand (40) einschließt, der sich von einem Umfang eines distalen Endes der proximalen Kapsel erstreckt; wobei der Rand mindestens ein röntgenopakes Element (44) einschließt.

9. System nach Anspruch 1, ferner umfassend einen Kolben (30), der innerhalb der flexiblen Kapsel beweglich ist.

10. System nach Anspruch 6, wobei die Herzklappenprothese eine Trikuspidalklappe ist.

11. System nach Anspruch 8, wobei der Rand eine Vielzahl von Armen (44) einschließt, die innerhalb der proximalen Kapsel positioniert sind.

12. System nach einem der vorstehenden Ansprüche, wobei die flexible Kapsel in dem ersten Zustand einen Winkel zwischen 90 und 170 Grad definiert.

## Revendications

1. Système comprenant :
un dispositif d'administration (10) comportant :
une gaine externe (24) ayant une partie distale rigide (26) ;
une gaine interne (20) positionnée au moins partiellement à l'intérieur de la gaine externe ; la gaine interne ayant une extrémité distale ;
un arbre (16) positionné coaxialement au moins partiellement à l'intérieur de la gaine externe et de la gaine interne ;
une capsule flexible (14) reliée à une extrémité distale de la gaine ; dans lequel la capsule flexible définit au moins partiellement un compartiment de prothèse (22) ; dans lequel la capsule flexible présente un premier état dans lequel un axe longitudinal (A) de la capsule flexible est incurvé ; et
dans lequel le dispositif d'administration présente un second état dans lequel la capsule flexible est positionnée à l'intérieur de la partie distale de la gaine externe de telle sorte que la rigidité de la partie distale de la gaine externe réduit la courbure de la capsule flexible, le dispositif d'administration présentant en outre un état dans lequel la capsule flexible est positionnée à l'extérieur de la gaine externe dans le premier état.

2. Système selon la revendication 1, dans lequel la capsule flexible est constituée d'un matériau à mémoire de forme rappelé vers le second état.

3. Système selon la revendication 2, dans lequel la capsule flexible comporte une pluralité de fentes (15).

4. Système selon la revendication 1, dans lequel la capsule flexible comporte une doublure intérieure (17).

5. Système selon la revendication 1, dans lequel la capsule flexible comporte une chemise extérieure (19).

6. Système selon la revendication 1, comprenant en outre une valvule cardiaque prothétique (12) supportée dans la capsule flexible.

7. Système selon la revendication 1, comprenant en outre une capsule proximale (18) reliée à l'extrémité distale de la gaine interne, dans lequel la capsule flexible et la capsule proximale définissent collectivement le compartiment de prothèse.

8. Système selon la revendication 7, dans lequel la capsule proximale comporte un rebord flexible (40) s'étendant depuis une circonférence d'une extrémité distale de la capsule proximale ; dans lequel le bord comprend au moins un élément radio-opaque (44).

9. Système selon la revendication 1, comprenant en outre un piston (30) mobile dans la capsule flexible.

10. Système selon la revendication 6, dans lequel la valvule cardiaque prothétique est une valvule tricuspide.

11. Système selon la revendication 8, dans lequel le bord comprend une pluralité de bras (44) positionnés dans la capsule proximale.

12. Système selon l'une quelconque revendication précédente, dans lequel la capsule flexible définit un angle compris entre 90 et 170 degrés dans le premier état.
